(19) Europäisches Patentamt
European Patent Office
Office européen des brevets

(11) EP 1 397 365 B1

(12) EUROPEAN PATENT SPECIFICATION

(45) Date of publication and mention of the grant of the patent: **16.02.2005 Bulletin 2005/07**

(21) Application number: **02724151.2**

(22) Date of filing: **23.05.2002**

(51) Int Cl.[7]: **C07D 487/08**, C07D 471/08, A61K 31/551, A61K 31/395, A61K 31/439, A61P 25/28, A61P 25/30, A61P 29/00

(86) International application number: **PCT/DK2002/000347**

(87) International publication number: **WO 2002/096911 (05.12.2002 Gazette 2002/49)**

# (54) HETEROARYL-DIAZABICYCLO-ALKANES AS CNS-MODULATORS

HETEROARYL-DIAZABICYCLOALKANDERIVATEN ALS CNS-MODULATOREN

HETEROARYL-DIAZABICYCLO-ALCANES A TITRE DE MODULATEURS DU SNC

(84) Designated Contracting States:
**AT BE CH CY DE DK ES FI FR GB GR IE IT LI LU MC NL PT SE TR**

(30) Priority: **01.06.2001 DK 200100866**

(43) Date of publication of application:
**17.03.2004 Bulletin 2004/12**

(73) Proprietor: **NeuroSearch A/S**
**2750 Ballerup (DK)**

(72) Inventors:
- **PETERS, Dan c/o NeuroSearch A/S DK-2750 Ballerup (DK)**
- **OLSEN, Gunnar M. c/o NeuroSearch A/S DK-2750 Ballerup (DK)**
- **NIELSEN, Elsebet, Östergaard c/o NeuroSearch A/S DK-2750 Ballerup (DK)**
- **AHRING, Philip, K. c/o NeuroSearch A/S DK-2750 Ballerup (DK)**
- **JöRGENSEN, Tino, Dyhring c/o NeuroSearch A/S DK-2750 Ballerup (DK)**
- **SLÖK, Frank, Abildgaard DK-3450 Alleroed (DK)**

(74) Representative: **Abildgren, Michael Padkjaer**
**NeuroSearch A/S**
**Patent Department**
**93 Pederstrupvej**
**2750 Ballerup (DK)**

(56) References cited:

| | |
|---|---|
| **EP-A- 0 406 439** | **WO-A-00/44755** |
| **WO-A-01/44243** | **WO-A-02/02564** |
| **WO-A-97/11945** | **WO-A-99/31100** |
| **WO-A-99/32487** | **US-A- 3 196 154** |
| **US-A- 5 478 939** | **US-A- 5 679 673** |
| **US-A1- 2002 013 309** | **US-A1- 2002 037 893** |
| **US-B1- 6 392 045** | |

- **BARLOCCO ET AL: "Mono- and disubstituted-3,8-diazabicyclo [3.2.1] octane derivatives as analgesics structurally related to epibatidine: Synthesis, activity, and modeling" JOURNAL OF MEDICINAL CHEMISTRY, AMERICAN CHEMICAL SOCIETY. WASHINGTON, US, vol. 41, no. 5, 1998, pages 674-681, XP002105020 ISSN: 0022-2623**
- **CHEMICAL ABSTRACTS, vol. 46, no. 10, 1952 Columbus, Ohio, US; abstract no. 992f, ZU-YOONG KYI ET AL: "Synthetic analgesics and related compounds. II. Some derivatives of 3,7-diazabicyclo[3.3.1]nonane (bispidine)." XP002902579 & J. CHEM. SOC., 1951, pages 1706-1708,**

EP 1 397 365 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

### TECHNICAL FIELD

The present invention relates to novel diazabicycloalkane derivatives, which are found to be cholinergic ligands at the nicotinic acetylcholine receptors and modulators of the monoamine receptors and transporters.

Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

### BACKGROUND ART

The present invention is devoted to the provision of modulators of the nicotinic receptor and/or of the monoamine receptors, which modulators are useful for the treatment of diseases or disorders related to the cholinergic receptors, and in particular the nicotinic acetyl choline receptor (nAChR), the monoamine receptors, in particular the serotonin receptor (5-HTR), the dopamine receptor (DAR) and the norepinephrine receptor (NER), and of the biogenic amine transporters for serotonin (5-HT), dopamine (DA) and norepinephrine (NE).

WO 0044755 discloses diazabicyclo derivatives useful as nicotinic acetylcholine ligands. However, the diazabicycloalkane derivatives of this invention are not disclosed, and no effect on monoamine reuptake is reported.

WO 0055143 discloses diazabicyclo derivatives useful as $\alpha$1-adrenoreceptor modulators. However, the diazabicycloalkane derivatives of this invention are not disclosed, and no effect on nicotinic receptors is reported.

WO 9711945 discloses diazabicyclo derivatives having selective 5-$HT_1$-like receptor antagonist actoivity. However, the diazabicycloalkane derivatives of this invention are not disclosed, and no effect on nicotinic receptors is reported.

EP 5468742 discloses diazabicyclo derivatives useful as antibacterial agents. However, the diazabicycloalkane derivatives of this invention are not disclosed, and no effect on monoamine reuptake or an nicotinic receptors is reported.

EP 5659038 discloses diazabicyclo derivatives useful as antibacterial agents. However, the diazabicycloalkane derivatives of this invention are not disclosed, and no effect on monoamine reuptake or on nicotinic receptors is reported.

### SUMMARY OF THE INVENTION

The present invention is devoted to the provision of novel modulators of the nicotinic receptor and/or of the monoamine receptors, which modulators are useful for the treatment of diseases or disorders related to the cholinergic receptors, and in particular the nicotinic acetyl choline receptor (nAChR), the monoamine receptors, in particular the serotonin receptor (5-HTR), the dopamine receptor (DAR) and the norepinephrine receptor (NER), and of the biogenic amine transporters for serotonin (5-HT), dopamine (DA) and norepinephrine (NE).

Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

The compounds of the invention may also be useful as diagnostic tools or monitoring agents in various diagnostic methods, and in particular for *in vivo* receptor imaging (neuroimaging), and they may be used in labelled or unlabelled form.

In its first aspect the invention provides a diazabicycloalkane derivative represented by Formula I,

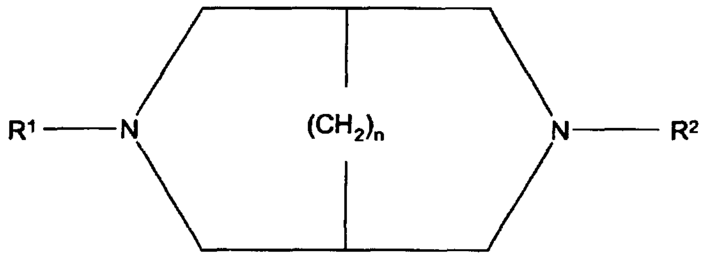

(I)

any of its enantiomers or any mixture of enantiomers, or a pharmaceutically acceptable salt thereof, wherein n represents 1, 2 or 3;

$R^1$ represents hydrogen or benzyl; and

$R^2$ representsa pyrazinyl or a pyridazinyl group, which heterocyclic group may be substituted one or more times with substituents selected from the group consisting of $C_{1-6}$-alkyl, $C_{1-6}$-alkoxy, halogen, $CF_3$, phenyl and naphthyl.

In another aspect the invention provides pharmaceutical compositions comprising a therapeutically effective amount of the diazabicycloalkane derivative of the invention, or a pharmaceutically acceptable addition salt thereof, together with at least one pharmaceutically acceptable carrier or diluent.

In a third aspect the invention relates to the use of the diazabicycloalkane derivative of the invention, or a pharmaceutically-acceptable addition salt thereof, for the manufacture of a pharmaceutical composition for the treatment, prevention or alleviation of a disease or a disorder or a condition of a mammal, including a human, which disease, disorder or condition is responsive to modulation of cholinergic receptors and/or monoamine receptors.

Other objects of the invention will be apparent to the person skilled in the art from the following detailed description and examples.

## DETAILED DISCLOSURE OF THE INVENTION

The present invention provides novel diazabicycloalkane derivatives represented by Formula I:

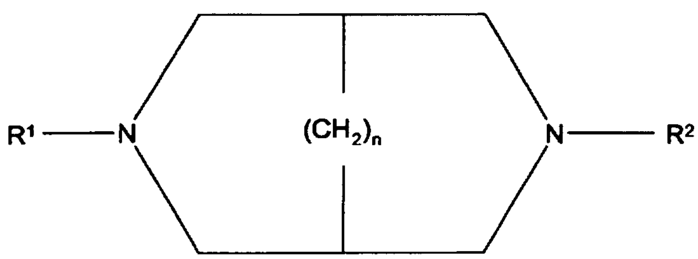

(I)

any of its enantiomers or any mixture of enantiomers, or a pharmaceutically acceptable salt thereof, wherein n represents 1, 2 or 3;

$R^1$ represents hydrogen or benzyl; and

$R^2$ representsa pyrazinyl or a pyridazinyl group, which heterocyclic group may be substituted one or more times with substituents selected from the group consisting of $C_{1-6}$-alkyl, $C_{1-6}$-alkoxy, halogen, $CF_3$, phenyl and naphthyl.

In a preferred embodiment $R^2$ represents a 2-pyrazinyl group, which is optionally substituted at position 5 or 6 with a substituent selected from the group consisting of $C_{1-6}$-alkyl, halogen, $CF_3$, phenyl and naphthyl.

In a most preferred embodiment the diazabicycloalkane derivative of the invention is

3-Benzyl-7-(6-chloro-2-pyrazinyl)-3,7-diazabicyclo-[3.3.1]-nonane; or

3-*H*-7-(6-Chloro-2-pyrazinyl)-3,7-diazabicyclo-[3.3.1]-nonane;

any of its enantiomers or any mixture of enantiomers, or a pharmaceutically acceptable salt thereof.

In another preferred embodiment $R^2$ represents a 3-pyridazinyl group, which is optionally substituted at position 5 or 6 with a substituent selected from the group consisting of $C_{1-6}$-alkyl, halogen, $CF_3$, phenyl and naphthyl.

In a most preferred embodiment the diazabicycloalkane derivative of the invention is 3-*H*-7-(6-phenyl-3-pyridazinyl)-3,7-diazabicyclo-[3.3.1]-nonane;

3-Benzyl-7-(6-phenyl-3-pyridazinyl)-3,7-diazabicyclo-[3.3.1]-nonane;
3-Benzyl-7-(6-chloro-3-pyridazinyl)-3,7-diazabicyclo-[3.3.1]-nonane;
3-*H*-7-(6-Chloro-3-pyridazinyl)-3,7-diazabicyclo-[3.3.1]-nonane;
3-Benzyl-7-(6-chloro-3-pyridazinyl)-3,7-diazabicyclo-[3.3.2]-decane; or
3-Benzyl-7-(6-chloro-3-pyridazinyl)-3,7-diazabicyclo-[3.3.3]-undecane;
any of its enantiomers or any mixture of enantiomers, or a pharmaceutically acceptable salt thereof.

Definition of Substituents

In the context of this invention halogen represents a fluorine, a chlorine, a bromine or an iodine atom. Thus, a trihalogenmethyl group represents e.g. a trifluoromethyl group and a trichloromethyl group.

In the context of this invention an alkyl group designates a univalent saturated, straight or branched hydrocarbon chain. The hydrocarbon chain preferably contain of from one to eighteen carbon atoms ($C_{1-18}$-alkyl), more preferred of from one to six carbon atoms ($C_{1-6}$-alkyl; lower alkyl), including pentyl, isopentyl, neopentyl, tertiary pentyl, hexyl and isohexyl. In a preferred embodiment alkyl represents a $C_{1-4}$-alkyl group, including butyl, isobutyl, secondary butyl, and tertiary butyl. In a preferred embodiment of this invention alkyl represents a $C_{1-3}$-alkyl group, which may in particular be methyl, ethyl, propyl or isopropyl.

In the context of this invention an alkoxy group designates an "alkyl-O-" group, wherein alkyl is as defined above.

Pharmaceutically Acceptable Salts

The diazabicycloalkane derivatives of the invention may be provided in any form suitable for the intended administration. Suitable forms include pharmaceutically (i.e. physiologically) acceptable salts, and pre- or prodrug forms of the chemical compound of the invention.

Examples of pharmaceutically acceptable addition salts include, without limitation, the non-toxic inorganic and organic acid addition salts such as the hydrochloride derived from hydrochloric acid, the hydrobromide derived from hydrobromic acid, the nitrate derived from nitric acid, the perchlorate derived from perchloric acid, the phosphate derived from phosphoric acid, the sulphate derived from sulphuric acid, the formate derived from formic acid, the acetate derived from acetic acid, the aconate derived from aconitic acid, the ascorbate derived from ascorbic acid, the benzenesulphonate derived from benzensulphonic acid, the benzoate derived from benzoic acid, the cinnamate derived from cinnamic acid, the citrate derived from citric acid, the embonate derived from embonic acid, the enantate derived from enanthic acid, the fumarate derived from fumaric acid, the glutamate derived from glutamic acid, the glycolate derived from glycolic acid, the lactate derived from lactic acid, the maleate derived from maleic acid, the malonate derived from malonic acid, the mandelate derived from mandelic acid, the methanesulphonate derived from methane sulphonic acid, the naphthalene-2-sulphonate derived from naphtalene-2-sulphonic acid, the phthalate derived from phthalic acid, the salicylate derived from salicylic acid, the sorbate derived from sorbic acid, the stearate derived from stearic acid, the succinate derived from succinic acid, the tartrate derived from tartaric acid, the toluene-p-sulphonate derived from p-toluene sulphonic acid, and the like. Such salts may be formed by procedures well known and described in the art.

Other acids such as oxalic acid, which may not be considered pharmaceutically acceptable, may be useful in the preparation of salts useful as intermediates in obtaining a diazabicycloalkane derivative of the invention and its pharmaceutically acceptable acid addition salt.

Metal salts of the diazabicycloalkane derivative of the invention include the alkali metal salts, such as the sodium salt of a chemical compound of the invention containing a carboxy group.

In the context of this invention the "onium salts" of N-containing compounds are also contemplated as pharmaceutically acceptable salts. Preferred "onium salts" include the alkyl-onium salts, the cycloalkyl-oniurn salts, and the cycloalkylalkyl-onium salts.

The diazabicycloalkane derivatives of the invention may be provided in dissoluble or indissoluble forms together with pharmaceutically acceptable solvents such as water, ethanol, and the like. Dissoluble forms may also include hydrated forms such as the monohydrate, the dihydrate, the hemihydrate, the trihydrate, the tetrahydrate, and the like. In general, the dissoluble forms are considered equivalent to indissoluble forms for the purposes of this invention.

Steric Isomers

The diazabicycloalkane derivatives of the present invention may exist in (+) and (-) forms as well as in racemic forms (±). The racemates of these isomers and the individual isomers themselves are within the scope of the present invention.

Racemic forms can be resolved into the optical antipodes by known methods and techniques. One way of separating the diastereomeric salts is by use of an optically active acid, and liberating the optically active amine compound by treatment with a base. Another method for resolving racemates into the optical antipodes is based upon chromatography on an optical active matrix. Racemic compounds of the present invention can thus be resolved into their optical antipodes, e.g., by fractional crystallisation of d- or l- (tartrates, mandelates, or camphorsulphonate) salts for example.

The diazabicycloalkane derivatives of the invention may also be resolved by the formation of diastereomeric amides by reaction of the chemical compounds of the present invention with an optically active activated carboxylic acid such as that derived from (+) or (-) phenylalanine, (+) or (-) phenylglycine, (+) or (-) camphanic acid or by the formation of diastereomeric carbamates by reaction of the chemical compound of the present invention with an optically active chloroformate or the like.

Additional methods for the resolving the optical isomers are known in the art. Such methods include those described by *Jaques J, Collet A, & Wilen S* in "Enantiomers, Racemates, and Resolutions", John Wiley and Sons, New York (1981).

Optical active compounds can also be prepared from optical active starting materials.

Prodrugs

The diazabicycloalkane derivatives of the invention may be administered as such or in the form of a suitable prodrug. The term "prodrug" denotes a bioreversible derivative of the drug, the bioreversible derivative being therapeutically substantially inactive per se but being able to convert in the body to the active substance by an enzymatic or non-enzymatic process.

Thus examples of suitable prodrugs of the diazabicycloalkane derivatives of the invention include compounds obtained by suitable bioreversible derivatization of one or more reactive or derivatizable groups of the parent substance to result in a bioreversible derivative. The derivatization may be performed to obtain a higher bioavailability of the active substance, to stabilize an otherwise unstable active substance, to increase the lipophilicity of the substance administered, etc.

Examples of types of chemical substances, which may advantageously be administered in the form of prodrugs, are carboxylic acids, other acidic groups and amines, which may be rendered more lipophilic by suitable bioreversible derivatization. Examples of suitable groups include bioreversible esters or bioreversible amides. Amino acids are typical examples of substances, which, in their unmodified form, may have a low absorption upon administration. Suitable prodrug derivatives of amino acids will be one or both of the above-mentioned types of bioreversible derivatives.

## Methods of Producing the Compounds

The diazabicycloalkane derivatives of the invention may be prepared by any conventional method useful for the preparation of analogous compounds and as described in the examples below.

Starting materials for the processes described herein are known or can be prepared by known processes from commercially available materials, e.g. as described in the working examples.

Also, one diazabicycloalkane derivative of the invention can be converted to another compound of the invention using conventional methods.

## Biological Activity

The diazabicycloalkane derivatives of the present are found to be cholinergic ligands at the nicotinic acetyl choline receptors (nAChR), and modulators of the monoamine receptors, in particular the biogenic amine transporters for serotonin (5-HT), dopamine (DA) and/or norepinephrine (NE).

In the context of this invention the term "modulator" covers agonists, partial agonists, antagonists and allosteric modulators of the particular receptor.

Due to their pharmacological profile the compounds of the invention may be useful for the treatment of diseases or conditions as diverse as CNS related diseases, PNS related diseases, diseases related to smooth muscle contraction, endocrine disorders, diseases related to neuro-degeneration, diseases related to inflammation, pain, and

withdrawal symptoms caused by the termination of abuse of chemical substances.

In a preferred embodiment the compounds of the invention are used for the treatment of diseases, disorders, or conditions relating to the central nervous system. Such diseases or disorders includes anxiety, cognitive disorders, learning deficit, memory deficits and dysfunction, Alzheimer's disease, attention deficit, attention deficit hyperactivity disorder, Parkinson's disease, Huntington's disease, Amyotrophic Lateral Sclerosis, Gilles de la Tourette's syndrome, depression, mania, manic depression, schizophrenia, obsessive compulsive disorders (OCD), panic disorders, eating disorders such as anorexia nervosa, bulimia and obesity, narcolepsy, nociception, AIDS-dementia, senile dementia, periferic neuropathy, autism, dyslexia, tardive dyskinesia, hyperkinesia, epilepsy, bulimia, post-traumatic syndrome, social phobia, sleeping disorders, pseudodementia, Ganser's syndrome, pre-menstrual syndrome, late luteal phase syndrome, chronic fatigue syndrome, mutism, trichotillomania, and jet-lag.

In another preferred embodiment the compounds of the invention may be useful for the treatment of diseases, disorders, or conditions associated with smooth muscle contractions, including convulsive disorders, angina pectoris, premature labour, convulsions, diarrhoea, asthma, epilepsy, tardive dyskinesia, hyperkinesia, premature ejaculation, and erectile difficulty.

In yet another preferred embodiment the compounds of the invention may be useful for the treatment of endocrine disorders, such as thyrotoxicosis, pheochromocytoma, hypertension and arrhythmias.

In still another preferred embodiment the compounds of the invention may be useful for the treatment of neurodegenerative disorders, including transient anoxia and induced neuro-degeneration.

In even another preferred embodiment the compounds of the invention may be useful for the treatment of inflammatory diseases, disorders, or conditions, including inflammatory skin disorders such as acne and rosacea, Chron's disease, inflammatory bowel disease, ulcerative colitis, and diarrhoea.

In still another preferred embodiment the compounds of the invention may be useful for the treatment of mild, moderate or even severe pain of acute, chronic or recurrent character, as well as pain caused by migraine, postoperative pain, and phantom limb pain.

Finally the compounds of the invention may be useful for the treatment of withdrawal symptoms caused by termination of use of addictive substances. Such addictive substances include nicotine containing products such as tobacco, opioids such as heroin, cocaine and morphine, benzodiazepines and benzodiazepine-like drugs, and alcohol. Withdrawal from addictive substances is in general a traumatic experience characterised by anxiety and frustration, anger, anxiety, difficulties in concentrating, restlessness, decreased heart rate and increased appetite and weight gain.

In this context "treatment" covers treatment, prevention, prophylactics and alleviation of withdrawal symptoms and abstinence as well as treatment resulting in a voluntary diminished intake of the addictive substance.

In another aspect, the compounds of the invention are used as diagnostic agents, e.g. for the identification and localisation of nicotinic receptors in various tissues. For this purpose the stannate derivatives of the invention are particularly useful.

**Neuroimaging**

The diazabicycloalkane derivatives of the invention, in particular those being selective for the nicotinic receptor subtype $\alpha 3$, $\alpha 4$ and/or $\alpha 7$ may be useful as diagnostic tools or monitoring agents in various diagnostic methods, and in particular for *in vivo* receptor imaging (neuroimaging).

In another aspect of the invention a method for the non-invasive determination of the distribution of a tracer compound inside a whole, intact living animal or human body using a physical detection method is provided. According to this method a tracer compound is a compound of the invention, or any of its enantiomers or any mixture of enantiomers, or a pharmaceutically acceptable salt thereof, in labelled or unlabelled form.

In a preferred embodiment the physical detection method is selected from PET, SPECT; MRS, MRI, CAT, or combinations thereof.

The compounds of the invention may be used in their labelled or unlabelled form. In the context of this invention "label" stands for the binding of a marker to the compound of interest that will allow easy quantitative detection of said compound.

The labelled compound of the invention preferably contains at least one radionuclide as a label. Positron emitting radionuclides are all candidates for usage. In the context of this invention the radionuclide is preferably selected from $^{11}C$, $^{18}F$, $^{15}O$, $^{13}N$, $^{123}I$, $^{125}I$, $^{131}I$, $^{3}H$ and $^{99m}$ Tc.

Examples of commercially available labelling agents, which can be used in the preparation of the labelled compounds of the present invention are $[^{11}C]O_2$, $^{18}F$ , and NaI with different isotopes of Iodine. In particular $[C^{11}]O_2$ may be converted to a $[^{11}C]$-methylating agent, such as $[^{11}C]H_3I$ or $[^{11}C]$-methyl triflate.

Labelled compounds containing e.g. $[^{125}I]$ labelled 1-iodoprop-1-en-3-yl as substituent on N-8 may be prepared as described in the art [*Elmaleh, et al.*; J. Nucl. Med. 1996 **37** 1197-1202].

Labelled compounds containing e.g. $[^{18}F]$-alkyl substituted N-8 may be prepared as described in the art, e.

g. in WO 96/39198.

The tracer compound can be selected in accordance with the detection method chosen.

in one preferred embodiment, the labelled or unlabelled compound of the invention can be detected by a suitable spectroscopic method, in particular UV spectroscopy and/or fluorescence spectroscopy.

In anther preferred embodiment, the compounds of the invention labelled by incorporation of a isotope into the molecule, which may in particular be an isotope of the naturally occurring atoms including deuterium, tritium, $^{13}C$, $^{14}C$, $^{131}I$, $^{125}I$, $^{123}I$, and $^{18}F$, the isotope incorporation may be measured by conventional scintillation counting techniques.

In a third preferred embodiment, the physical method for detecting said tracer compound of the present invention is selected from Position Emission Tomography (PET), Single Photon Imaging Computed Tomography (SPECT), Magnetic Resonance Spectroscopy (MRS), Magnetic Resonance Imaging (MRI), and Computed Axial X-ray Tomography (CAT), or combinations thereof.

Before conducting the method of the present invention, a diagnostically effective amount of a labelled or unlabelled compound of the invention is administered to a living body, including a human.

The diagnostically effective amount of the labelled or unlabelled compound of the invention to be administered before conducting the in-vivo method for the present invention is within a range of from 0.1 ng to 100 mg per kg body weight, preferably within a range of from 1 ng to 10 mg per kg body weight.

**Pharmaceutical Compositions**

In another aspect the invention provides novel pharmaceutical compositions comprising a therapeutically effective amount of the diazabicycloalkane derivative of the invention.

While a diazabicycloalkane derivative of the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising the diazabicycloalkane derivative of the invention, or a pharmaceutically acceptable salt or derivative thereof, together with one or more pharmaceutically acceptable carriers therefore, and, optionally, other therapeutic and/or prophylactic ingredients, know and used in the art. The carrier(s) must be "acceptable" in the sense of being compatible with the other ingredients of the formulation and not harmful to the recipient thereof.

Pharmaceutical compositions of the invention may be those suitable for oral, rectal, bronchial, nasal, topical (including buccal and sub-lingual), transdermal, vaginal or parenteral (including cutaneous, subcutaneous, intramuscular, intraperitoneal, intravenous, intraarterial, intracerebral, intraocular injection or infusion) administration, or those in a form suitable for administration by inhalation or insufflation, including powders and liquid aerosol administration, or by sustained release systems. Suitable examples of sustained release systems include semipermeable matrices of solid hydrophobic polymers containing the compound of the invention, which matrices may be in form of shaped articles, e.g. films or microcapsules.

The diazabicycloalkane derivative of the invention, together with a conventional adjuvant, carrier, or diluent, may thus be placed into the form of pharmaceutical compositions and unit dosages thereof. Such forms include solids, and in particular tablets, filled capsules, powder and pellet forms, and liquids, in particular aqueous or non-aqueous solutions, suspensions, emulsions, elixirs, and capsules filled with the same, all for oral use, suppositories for rectal administration, and sterile injectable solutions for parenteral use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

The diazabicycloalkane derivative of the present invention can be administered in a wide variety of oral and parenteral dosage forms. It will be obvious to those skilled in the art that the following dosage forms may comprise, as the active component, either a chemical compound of the invention or a pharmaceutically acceptable salt of a chemical compound of the invention.

For preparing pharmaceutical compositions from a diazabicycloalkane derivative of the present invention, pharmaceutically acceptable carriers can be either solid or liquid. Solid form preparations include powders, tablets, pills, capsules, cachets, suppositories, and dispersible granules. Solid carriers can be one or more substances, which may also act as diluents, flavouring agents, solubilizers, lubricants, suspending agents, binders, preservatives, tablet disintegrating agents, or an encapsulating material.

In powders, the carrier is a finely divided solid, which is in a mixture with the finely divided active component.

In tablets, the active component is mixed with the carrier having the necessary binding capacity in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatine, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as a mixture of fatty acid glyceride or cocoa butter, is first melted and the active component is dispersed homogeneously therein, as by stirring. The molten homogenous mixture is then poured into convenient sized moulds, allowed to cool, and thereby to solidify.

Compositions suitable for vaginal administration may be presented as pessaries, tampons, creams, gels, pastes, foams or sprays containing in addition to the active ingredient such carriers as are known in the art to be appropriate.

Liquid preparations include solutions, suspensions, and emulsions, for example, water or water-propylene glycol solutions. For example, parenteral injection liquid preparations can be formulated as solutions in aqueous polyethylene glycol solution.

The diazabicycloalkane derivative of the present invention may thus be formulated for parenteral administration (e.g. by injection, for example bolus injection or continuous infusion) and may be presented in unit dose form in ampoules, prefilled syringes, small volume infusion or in multi-dose containers with an added preservative. The compositions may take such forms as suspensions, solutions, or emulsions in oily or aqueous vehicles, and may contain formulation agents such as suspending, stabilising and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous solutions suitable for oral use can be prepared by dissolving the active component in water and adding suitable colorants, flavours, stabilising and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations, which preparations are intended converted shortly before use, to liquid form preparations for oral administration. Such liquid forms include solutions, suspensions, and emulsions. These preparations may contain, in addition to the active component, colorants, flavours, stabilisers, buffers, artificial and natural sweeteners, dispersants, thickeners, solubilizing agents, and the like.

For topical administration to the epidermis the diazabicycloalkane derivative may be formulated as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also contain one or more emulsifying agents, stabilising agents, dispersing agents, suspending agents, thickening agents, or colouring agents.

Compositions suitable for topical administration in the mouth include lozenges comprising the active agent in a flavoured base, usually sucrose and acacia or tragacanth; pastilles comprising the active ingredient in an inert base such as gelatine and glycerine or sucrose and acacia; and mouthwashes comprising the active ingredient in a suitable liquid carrier.

Solutions or suspensions are applied directly to the nasal cavity by conventional means, for example with a dropper, pipette or spray. The compositions may be provided in single or multi-dose form. In the latter case of a dropper or pipette this may be achieved by the patient administering an appropriate, predetermined volume of the solution or suspension. In the case of a spray, this may be achieved for example by means of a metering atomising spray pump.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatine, or blister packs from which the powder may be administered by means of an inhaler.

In compositions intended for administration to the respiratory tract, including intranasal compositions, the compound will generally have a small particle size for example of the order of 5 microns or less. Such a particle size may be obtained by means known in the art, for example by micronization.

When desired, compositions adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packaged tablets, capsules, and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration and continuous infusion are preferred compositions.

Further details on techniques for formulation and administration may be found in the latest edition of Remington's Pharmaceutical Sciences (Maack Publishing Co., Easton, PA).

A therapeutically effective dose refers to that amount of active ingredient, which ameliorates the symptoms or condition. Therapeutic efficacy and toxicity, e.g. $ED_{50}$ and $LD_{50}$, may be determined by standard pharmacological procedures in cell cultures or experimental animals. The dose ratio between therapeutic and toxic effects is the therapeutic index and may be expressed by the ratio $LD_{50}/ED_{50}$. Pharmaceutical compositions exhibiting large therapeutic indexes are preferred.

The dose administered must of course be carefully adjusted to the age, weight and condition of the individual being treated, as well as the route of administration, dosage form and regimen, and the result desired, and the exact dosage should of course be determined by the practitioner.

The actual dosage depend on the nature and severity of the disease being treated and the route of administration, and is within the discretion of the physician, and may be varied by titration of the dosage to the particular circumstances of this invention to produce the desired therapeutic effect. However, it is presently contemplated that pharmaceutical compositions containing of from about 0.01 to about 500 mg of active ingredient per individual dose, preferably of from about 0.1 to about 100 mg, most preferred of from about 1 to about 10 mg, are suitable for therapeutic treatments.

The active ingredient may be administered in one or several doses per day. A satisfactory result can, in certain instances, be obtained at a dosage as low as 0.01 µg/kg i.v. and 0.1 µg/kg p.o. The upper limit of the dosage range is presently considered to be about 10 mg/kg i.v. and 100 mg/kg p.o. Preferred ranges are from about 0.1 µg/kg to about 10 mg/kg/day i.v., and from about 1 µg/kg to about 100 mg/kg/day p.o.

## EXAMPLES

The invention is further illustrated with reference to the following examples, which are not intended to be in any way limiting to the scope of the invention as claimed.

### Example 1

#### General

All reactions involving air sensitive reagents or intermediates are performed under nitrogen and in anhydrous solvents. Magnesium sulphate is used as drying agent in the workup-procedures and solvents are evaporated under reduced pressure. The products are normally isolated as salts by stirring the free base with an excessive amount of a saturated solution of fumaric acid salt in a mixture of methanol and diethyl ether (1:9).

#### Starting materials

3,7-Dibenzyl-3,7-diazabicyclo-[3.3.1]-nonan-9-one;

Was prepared according to *Garrison GL;* J. Org. Chem. 1993 **58** 7670 - 7678.

3-Benzyl-7*H*-3,7-diazabicyclo-[3.3.2]-decane

The title compound is prepared from 3,7-dibenzyl-3.7-diazabicyclo-[3.3.2]-decane according to method B.

3,7-Dibenzyl-3,7-diazabicyclo-[3.3.2]-decane

The title compound is prepared by LiAlH4-reduction of 3,7-Dibenzyl-3.7-diazabicyclo-[3.3.2]-decane-2,6-dione (see *Wood G and Woo EP;* Canadian Journal of Chemistry 1968 **46** 3713-3717).

3,7-Dibenzyl-3,7-diazabicyclo-[3.3.2]-decane-2,6-dione

The title compound is prepared by a double Schmidt reaction from bicyclo-[2.2.2]-octane-2,5-dione using BzN3 (see e.g. *Desai P et. al.*; J. Am. Chem. Soc. 2000 **122** 7226-7232, and *Alvarez* SG *and Alvarez MT*; Synthesis 1997 413-414) as reagent to achieve the right regiochemistry.

2.8-Diazabicyclo-[3.3.3]-undecane; and

2.6-diazabicyclo-[3.3.3]-undecane

The title compound is prepared by a double Schmidt reaction from bicyclo-[3.2.2]-octane-6,8-dione using sodium azide as reagent to achieve the right regiochemistry.

2,8-Diazabicyclo-[3.3.2]-decane; and

2,6-diazabicyclo-[3.3.2]-decane

The title compound is prepared by a double Schmidt reaction from bicyclo-[2.2.2]-octane-2,5-dione using sodium azide as reagent to achieve the right regiochemistry.

5,7-dioxobicyclo-[2.2.2]-oct-2-ene

The title compound is as described by *Hill RK et. al.*, J. Org. Chem. 1985 **50** 5528-5533.

**Method A**

3,7-Dibenzyl-3,7-diazabicyclo-[3.3.1]-nonane (Intermediate)

A mixture of 3,7-dibenzyl-3,7-diazabicyclo-[3.3.1]-nonan-9-one (13.2 g, 41.3 mmol), potassium hydroxide (13.9 g, 248 mmol), hydrazine hydrate (15.4 ml, 496 mmol), diethylene glycol (200 ml) and mesitylene (300 ml) was heated at 200°C with a Dean & Stark water collector overnight.
Aqueous sodium hydroxide (400 ml, 1 M) was added, and the mixture was extracted with diethyl ether (2 x 300 ml). The organic phase was back extracted with sodium hydroxide (300 ml, 1 M) to remove diethylene glycol.
The product (verified by nmr and GC-MS) was isolated as an oil: 12.65 g (100%).

**Method B**

3-Benzyl-7-*H*-3,7-diazabicyclo-[3.3.1]-nonane (Intermediate)

A stirred mixture of 3,7-Dibenzyl-3,7-diazabicyclo-[3.3.1]-nonane (16.3 g, 52.1 mmol) and methanol (150 ml) was equilibrated with an atmosphere of nitrogen. Formic acid (13.76 ml, 365 mmol) and palladium on carbon (5.0 g, 10%) was added. The mixture was stirred at room temperature overnight.
Aqueous sodium hydroxide (300 ml, 1M) was added and the mixture was extracted with ethyl acetate (2 x 200 ml).
Pure product (verified by nmr and GC-MS) was isolated as an oil (6.5 g, 57%).

3-*H*-7-(2-Quinolinyl)-3,7-diazabicyclo-[3.3.1]-nonane fumaric acid salt (Compound B1)

Was prepared according to method B from 3-Benzyl-7-(2-quinolinyl)-3.7-diazabicyclo-[3.3.1]-nonane. Mp. 147.0-153.0°C.

3-*H*-7-(6-phenyl-3-pyridazinyl)-3,7-diazabicyclo-[3.3.1]-nonane fumaric acid salt (Compound B2)

Was prepared according to method B from 3-benzyl-7-(6-phenyl-3-pyridazinyl)-3.7-diazabicyclo-[3.3.1]-nonane. Mp 177.5-180.7°C.

3-*H*-7-*tert*-butoxycarbonyl-3,7-diazabicyclo-[3.3.1]-nonane (Intermediate)

Was prepared from 3-benzyl-7-*tert*-butoxycarbonyl-3,7-diazabicyclo-[3.3.1]-nonane according to method B.

**Method C**

3-Benzyl-7-(6-phenyl-3-pyridazinyl)-3,7-diazabicyclo-[3.3.1]-nonane fumaric acid salt (Compound C1)

A mixture of 3-benzyl-7-*H*-3,7-diazabicyclo-[3.3.1]-nonane (2.0 g, 9.2 mmol), 3-chloro-6-phenyl-pyridazine (5.3 g, 27.6 mmol) and 1,4-dioxane (5 ml) was stirred at 100 °C for 90 minutes. Aqueous sodium hydroxide (50 ml, 1 M) was added and the mixture was extracted with dichloromethane (2 x 50 ml).

The crude mixture was purified by silica gel column chromatography, using a mixture of heptane and ethyl acetate (1:2) as solvent. The corresponding salt was obtained by addition of a diethyl ether and methanol mixture (9: 1), saturated with fumaric acid. Mp. 172.5-174°C.

3-Benzyl-7-(4-methyl-2-quinolinyl)-3,7-diazabicyclo-[3.3.1]-nonane (Compound C2)

Was prepared according to method C.

3-Benzyl-7-(2-quinolinyl)-3,7-diazabicyclo-[3.3.1]-nonane fumaric acid salt (Compound C3)

Was prepared according to method C. Mp. 56.1-56.8°C.

3-Benzyl-7-(6-chloro-3-pyridazinyl)-3,7-diazabicyclo-[3.3.1]-nonane fumaric acid salt (Compound C4)

Was prepared according to method C from 3,6-dichloropyridazine. Mp. 204-205°C.

3-Benzyl-7-(6-chloro-2-pyrazinyl)-3,7-diazabicyclo-[3.3.1]-nonane fumaric acid salt (Compound C5)

Was prepared according to method C from 2,6-dichloropyrazine. Mp. 183.1-183.3°C.

3-Benzyl-7-(6-nitro-2-quinolinyl)-3,7-diazabicyclo-[3.3.1]-nonane fumaric acid salt (Compound C6)

Was prepared according to method C from 2-chloro-6-nitroquinoline. Mp. 168-172°C.

3-Benzyl-8-(6-chloro-3-pyridazinyl)-3,8-diazabicyclo-[4.3.1]-decane fumaric acid salt (Compound C7); and

8-Benzyl-3-(6-chloro-3-pyridazinyl)-3,8-diazabicyclo-[4.3.1]-decane fumaric acid salt (Compound C8)

The title compounds are prepared using the sequence described above: Starting from 3,6-dichloropyridazine and 3,8-dibenzyl-3.8-diazabicyclo-[4.3.1]-decane, from 3,8-dibenzyl-3.8-diazabicyclo-[4.3.1]-decan-10-one using *N*-benzyl-homo-4-piperidone in the same manner as *N*-benzyl-4-piperidone as described above.

3-Benzyl-7-(6-chloro-3-pyridazinyl)-3,7-diazabicyclo-[3.3.2]-decane (Compound C9)

The title compound is prepared from 3-benzyl-7*H*-3,7-diazabicyclo-[3.3.2]-decane and 3,6-dichloropyridazine according to method C.

3-Benzyl-7-(6-chloro-3-pyridazinyl)-3,7-diazabicyclo-[3.3.3]-undecane (Compound C10)

The title compound is prepared as described for 3-benzyl-7-(6-chloro-3-pyridazinyl)-3,7-diazabicyclo-[3.3.2]-decane starting from the higher homologe bicyclo-[3.2.2]-nonane-6,8-dione.

8-(6-Chloro-3-pyridazinyl)-2-*H*-2,8-diazabicyclo-[3.3.3]-undecane (Compound C11)

The title compound is prepared from 2,8-diazabicyclo-[3.3.3]-undecane according to method C.

6-(6-Chloro-3-pyridazinyl)-2-*H*-2,6-diazabicyclo-[3.3.3]-undecane (Compound C12)

The title compound is prepared from 2,8-diazabicyclo-[3.3.3]-undecane according to method C.

8-(6-Chloro-3-pyridazinyl)-2-*H*-2,8-diazabicyclo-[3.3.2]-decane (Compound C13)

The title compound is prepared from 2,8-diazabicyclo-[3.3.2]-decane according to method C.

6-(6-Chloro-3-pyridazinyl)-2-*H*-2,6-diazabicyclo-[3.3.2]-decane (Compound C14)

The title compound is prepared from 2,6-diazabicyclo-[3.3.2]-decane according to method C.

**Method D**

3-Methyl-7-(6-phenyl-3-pyridazinyl)-3,7-diazabicyclo-[3.3.1]-nonane fumaric acid salt (Compound D1)

A mixture of 3-*H*-7-(6-phenyl-3-pyridazinyl)-3,7-diazabicyclo-[3.3.1]-nonane (0.50 g, 3.6mmol), conc. formic acid (5 ml) and formaldehyde (5 ml) was stirred at reflux for 1.5 hours. The mixture was evaporated. Aqueous sodium hydroxide (50 ml, 1 M) was added and the mixture was extracted with dichloromethane (2 x 50 ml). The corresponding salt was obtained by addition of a diethyl ether and methanol mixture (9:1), saturated with fumaric acid. Mp. 213.9-220.8°C.

3-Methyl-7-(2-quinolinyl)-3,7-diazabicyclo-[3.3.1]-nonane fumaric acid salt (Compound D2)

Was prepared from 3-*H*-7-(2-quinolinyl)-3,7-diazabicyclo-[3.3.1]-nonane according to method D

3-Allyl-7-(6-phenyl-3-pyridazinyl)-3,7-diazabicyclo-[3.3.1]-nonane fumaric acid salt (Compound D3)

A mixture of 3-*H*-7-(6-phenyl-3-pyridazinyl)-3,7-diazabicyclo-[3.3.1]-nonane (0.50 g, 1.8 mmol), potassium carbonate (0.25 g, 1.8 mmol), allyl bromide (0.17 ml, 1.8 mmol) and DMF (5 ml) was stired at 80°C. Aqueous sodium hydroxide (20 ml, 1 M) was added and the mixture was extracted with dichloromethane (2 x 10 ml). The crude mixture was purified by silica gel column chromatography, using a mixture of dichloromethane, methanol, aqueous ammonia (89, 10, 1) as solvent. The corresponding salt was obtained by addition of a diethyl ether and methanol mixture (9:1), saturated with fumaric acid. Yield 110 mg Mp. 183.5-187.6°C.

**Method E**

3-*Tert*-butoxycarbonyl-7-(6-chloro-3-pyridazinyl)-3,7-diazabicyclo-[3.3.1]-nonane (Intermediate)

A mixture of 3-*H*-7-*tert*-butoxycarbonyl-3,7-diazabicyclo-[3.3.1]-nonane (2.0 g, 8.8 mmol), 3,6-didichloropyridazine (2.6 g, 17.7 mmol) and dioxane (5 ml) was stirred at 90°C for 3 hours. The crude mixture was purified by silica gel column chromatography, using a mixture of heptane and ethyl acetate (1:2) as solvent. Yield 380 mg (13%).

3-*Tert*-butoxycarbonyl-7-(6-chloro-2-pyrazinyl)-3,7-diazabicyclo-[3.3.1]-nonane (Intermediate)

Was prepared according to method E.

**Method F**

3-*H*-7-(6-Chloro-3-pyridazinyl)-3,7-diazabicyclo-[3.3.1]-nonane fumaric acid salt (Compound F1)

A mixture of 3-*tert*-butoxycarbonyl-7-(6-chloro-3-pyridazinyl)-3,7-diazabicyclo-[3.3.1]-nonane (0.38 g, 1.1 mmol), trifluoroacetic acid (1.7 ml) and dichloromethane (5 ml) was stirred at room temperature for 2 hours. Aqueous sodium hydroxide (50 ml, 1 M) was added and the mixture was extracted with dichloromethane (2 x 50 ml). The crude mixture was purified by silica gel column chromatography, using a mixture of dichloromethane, methanol, aqueous ammonia (89:10:1) as solvent. The corresponding salt was obtained by addition of a diethyl ether and methanol mixture (9:1), saturated with fumaric acid. Yield 110 mg. Mp. 195.5-196.5°C.

3-*H*-7-(6-Chloro-2-pyrazinyl)-3,7-diazabicyclo-[3.3.1]-nonane fumaric acid salt (Compound F2)

Was prepared according to method F. Mp. 180.3-181.9°C.

3-Benzyl-7-*tert*-butoxycarbonyl-3,7-diazabicyclo-[3.3.1]-nonane (Intermediate)

A mixture of 3-benzyl-7-*H*-3,7-diazabicyclo-[3.3.1]-nonane (6.8 g, 31.2 mmol), aqueous, saturated sodium hydrogencarbonate (83 ml, 94 mmol), *tert*-butoxycarbonylanhydride (6.83 g, 31.2 mmol) and dichloromethane (80 ml) was stirred at room temperature for 3 hours. The organic phase was separated and was washed with water (50 ml). Yield 9.9 g (100%).

**Example 2**

***In vitro* Inhibition of $^3$H-5-Hydroxytryptamine ($^3$H-5-HT, Serotonin) Uptake in Cortical Synaptosomes**

Serotonin transporters/uptake sites on nerve terminals presumably function to terminate neuronal signalling by removing serotonin from the synaptic cleft. The activity of the serotonin transporter integral protein can be measured *in vitro* by synaptosomal uptake of $^3$H-5-hydroxytryptamine.

Preparations are performed at 0-4°C unless otherwise indicated. Cerebral cortices from male Wistar rats (150-200 g) are homogenized for 5-10 sec in 100 volumes of ice-cold 0.32M sucrose containing 1 mM pargyline using a motor driven teflon pestle in a glass homogenizing vessel. Monoamine oxidase activity will be inhibited in the presence of pargyline. The homogenate is centrifuged at 1000 x g for 10 min. The resulting supernatant is then centrifuged at 27,000 x g for 50 min and the supernatant is discarded. The pellet ($P_2$) is re-suspended in oxygenated (equilibrated with an atmosphere of 96% $O_2$: 4% $CO_2$ for at least 30 min) Krebs-Ringer incubation buffer (1000 ml per g of original tissue) at pH 7.2 containing 122 mM NaCl, 0.16 mM EDTA, 4.8 mM KCl, 12.7 mM $Na_2HPO_4$, 3.0 mM $NaH_2PO_4$, 1.2 mM $MgSO_4$, 1 mM $CaCl_2$, 10 mM glucose and 1 mM ascorbic acid.

Aliquots of 4.0 ml tissue suspension are added to 100 µl of test solution and 100 µl of $^3$H-5-HT (1 nM, final concentration), mixed and incubated for 30 min at 37°C. Non-specific uptake is determined using citalopram (1 µM, final concentration). After incubation the samples are poured directly onto Whatman GF/C glass fibre filters under suction. The filters are then washed three times with 5 ml of ice-cold 0.9% (w/v) NaCl solution. The amount of radioactivity on the filters is determined by conventional liquid scintillation counting. Specific uptake is calculated as the difference between total uptake and non-specific uptake.

25-75% inhibition of specific binding must be obtained, before calculation of an $IC_{50}$.

The test value is given as $IC_{50}$ (the concentration (µM) of the test substance which inhibits the specific binding of $^3$H-5-HT by 50%).

$$IC_{50} = \text{(applied test substance concentration, µM)} \times \frac{1}{\left(\frac{C_o}{C_x} - 1\right)}$$

where $C_0$ is specific binding in control assays and $C_x$ is the specific binding in the test assay (the calculations assume normal mass-action kinetics).

The results are presented in Table 1 below.

Table 1

| *In vitro* Inhibition of $^3$H-5-Hydroxytryptamine | | |
|---|---|---|
| **Compound** | **Compound No.** | **$IC_{50}$ (µM)** |
| 3-Benzyl-7-(2-quinolinyl)-3,7-diazabicyclo-[3.3.1]-nonane | C3 | 0.022 |
| 3-Benzyl-7-(6-nitro-2-quinolinyl)-3,7-diazabicyclo-[3.3.1]-nonane | C6 | 0.0057 |

**Example 3**

***In vitro* Inhibition of $^3$H-Cytisine Binding**

Molecular biology studies have elucidated that there are at least ten nicotinic receptor genes in the brain. The predominant subtype with high affinity for nicotine is comprised of $\alpha_4$ and $\beta_2$ subunits. nAChRs of the latter type can selectively be labelled by the nicotine agonist $^3$H-cytisine.

Preparations are performed at 0-4°C. Cerebral corticies from male Wistar rats (150-250 g) are homogenized for 20 sec in 15 ml Tris, HCl (50 mM, pH 7.4) containing 120 mM NaCl, 5 mM KCl, 1 mM $MgCl_2$ and 2.5 mM $CaCl_2$ using an Ultra-Turrax homogenizer. The homogenate is centrifuged at 27,000 x g for 10 min. The supernatant is discarded and the pellet is resuspended in fresh buffer and centrifuged a second time. The final pellet is resuspended in fresh buffer (35 ml per g of original tissue) and used for binding assays.

Aliquots of 500 µl homogenate are added to 25 µl of test solution and 25 µl of $^3$H-cytisine (1 nM, final concentration), mixed and incubated for 90 min at 2°C. Non-specific binding is determined using (-)-nicotine (100 µM, final concentration). After incubation the samples are added 5 ml of ice-cold buffer and poured directly onto Whatman GF/C glass fibre filters under suction and immediately washed with 2 x 5 ml ice-cold buffer. The amount of radioactivity on the filters is determined by conventional liquid scintillation counting. Specific binding is total binding minus non-specific binding.

The test value is given as an $IC_{50}$ (the concentration (µM) of the test substance which inhibits the specific binding of $^3$H-cytisine by 50%).

The $IC_{50}$ value is determined from the inhibition curve. If a full curve is not available a 25-75% inhibition of specific binding must be obtained, before calculation of an $IC_{50}$.

$$IC_{50} = \text{(applied test substance concentration, µM)} \times \frac{1}{\left(\frac{C_o}{C_x} - 1\right)}$$

where $C_0$ is specific binding in control assays and $C_x$ is the specific binding in the test assay (the calculations assume normal mass-action kinetics).

The results are presented in Table 2 below.

Table 2

| *In vitro* Inhibition of $^3$H-cytisine Binding | | |
|---|---|---|
| **Compound** | **Compound No.** | **$IC_{50}$ (µM)** |
| 3-H-7-(6-Chloro-3-pyridazinyl)-3,7-diazabicyclo-[3.3.1]-nonane | F1 | 0.0030 |
| 3-H-7-(6-Chloro-2-pyrazinyl)-3,7-diazabicyclo-[3.3.1]-nonane | F2 | 0.0034 |

**Claims**

**1.** A diazabicycloalkane derivative represented by Formula I,

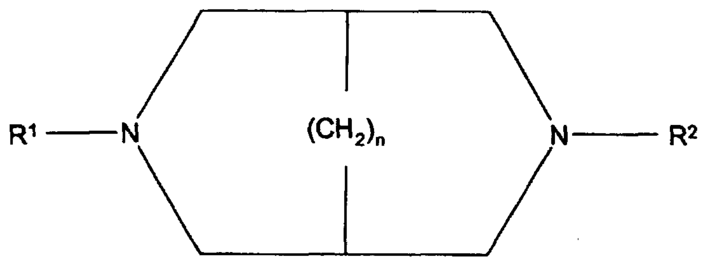

(I)

any of its enantiomers or any mixture of enantiomers, or a pharmaceutically acceptable salt thereof, wherein
n represents 1, 2 or 3;
$R^1$ represents hydrogen or benzyl; and
$R^2$ represents a pyrazinyl or a pyridazinyl group, which heterocyclic group may be substituted one or more times with substituents selected from the group consisting of $C_{1-6}$-alkyl, $C_{1-6}$-alkoxy, halogen, $CF_3$, phenyl and naphthyl.

**2.** The diazabicycloalkane derivative of claim 1, wherein $R^2$ represents a 2-pyrazinyl group, which is optionally substituted at position 5 or 6 with a substituent selected from the group consisting of $C_{1-6}$-alkyl, halogen, $CF_3$, phenyl and naphthyl.

**3.** The diazabicycloalkane derivative of claim 2, which is
3-Benzyl-7-(6-chloro-2-pyrazinyl)-3,7-diazabicyclo-[3.3.1]-nonane; or
3-*H*-7-(6-Chloro-2-pyrazinyl)-3,7-diazabicyclo-[3.3.1]-nonane;
any of its enantiomers or any mixture of enantiomers, or a pharmaceutically acceptable salt thereof.

**4.** The diazabicycloalkane derivative of claim 1, wherein $R^2$ represents a 3-pyridazinyl group, which is optionally substituted at position 5 or 6 with a substituent selected from the group consisting of $C_{1-6}$-alkyl, halogen, $CF_3$, phenyl and naphthyl.

**5.** The diazabicycloalkane derivative of claim 4, which is
3-*H*-7-(6-phenyl-3-pyridazinyl)-3,7-diazabicyclo-[3.3.1]-nonane;
3-Benzyl-7-(6-phenyl-3-pyridazinyl)-3,7-diazabicyclo-[3.3.1]-nonane;
3-Benzyl-7-(6-chloro-3-pyridazinyl)-3,7-diazabicyclo-[3.3.1]-nonane;
3-*H*-7-(6-Chloro-3-pyridazinyl)-3,7-diazabicyclo-[3.3.1]-nonane;
3-Benzyl-7-(6-chloro-3-pyridazinyl)-3,7-diazabicyclo-[3.3.2]-decane; or
3-Benzyl-7-(6-chloro-3-pyridazinyl)-3,7-diazabicyclo-[3.3.3]-undecane;
any of its enantiomers or any mixture of enantiomers, or a pharmaceutically acceptable salt thereof.

**6.** A pharmaceutical composition comprising a therapeutically effective amount of the diazabicycloalkane derivative of any one of claims 1-5, or a pharmaceutically acceptable addition salt thereof, together with at least one pharmaceutically acceptable carrier or diluent.

**7.** The use of the diazabicycloalkane derivative according to any one of claims 1-5, or a pharmaceutically-acceptable addition salt thereof, for the manufacture of a pharmaceutical composition for the treatment, prevention or alleviation of a disease or a disorder or a condition of a mammal, including a human, which disease, disorder or condition is responsive to modulation of cholinergic receptors and/or monoamine receptors.

**8.** The use according to claim 7, wherein the disease, disorder or condition relates to the central nervous system.

**9.** The use according to claim 7, wherein the disease, disorder or condition is anxiety, cognitive disorders, leaming deficit, memory deficits and dysfunction, Alzheimer's disease, attention deficit, attention deficit hyperactivity disorder, Parkinson's disease, Huntington's disease, Amyotrophic Lateral Sclerosis, Gilles de la Tourette's syndrome, depression, mania, manic depression, schizophrenia, obsessive compulsive disorders (OCD), panic disorders, eating disorders such as anorexia nervosa, bulimia and obesity, narcolepsy, nociception, AIDS-dementia, senile

dementia, periferic neuropathy, autism, dyslexia, tardive dyskinesia, hyperkinesia, epilepsy, bulimia, post-traumatic syndrome, social phobia, sleeping disorders, pseudodementia, Ganser's syndrome, pre-menstrual syndrome, late luteal phase syndrome, chronic fatigue syndrome, mutism, trichotillomania, and jet-lag.

**10.** The use according to claim 7, wherein the disease, disorder or condition are associated with smooth muscle contractions, including convulsive disorders, angina pectoris, premature labour, convulsions, diarrhoea, asthma, epilepsy, tardive dyskinesia, hyperkinesia, premature ejaculation, and erectile difficulty.

**11.** The use according to claim 7, wherein the disease, disorder or condition is related to the endocrine system, such as thyrotoxicosis, pheochromocytoma, hypertension and arrhythmias.

**12.** The use according to claim 7, wherein the disease, disorder or condition is a neurodegenerative disorders, including transient anoxia and induced neuro-degeneration.

**13.** The use according to claim 7, wherein the disease, disorder or condition is an inflammatory disorder, including inflammatory skin disorders such as acne and rosacea, Chron's disease, inflammatory bowel disease, ulcerative colitis, and diarrhoea.

**14.** The use according to claim 7, wherein the disease, disorder or condition is mild, moderate or even severe pain of acute, chronic or recurrent character, as well as pain caused by migraine, postoperative pain, and phantom limb pain.

**15.** The use according to claim 7, wherein the disease, disorder or condition is associated with withdrawal symptoms caused by termination of use of addictive substances, including nicotine containing products such as tobacco, opioids such as heroin, cocaine and morphine, benzodiazepines and benzodiazepine-like drugs, and alcohol.

**Patentansprüche**

**1.** Diazabicycloalkanderivat, wiedergegeben durch die Formel I,

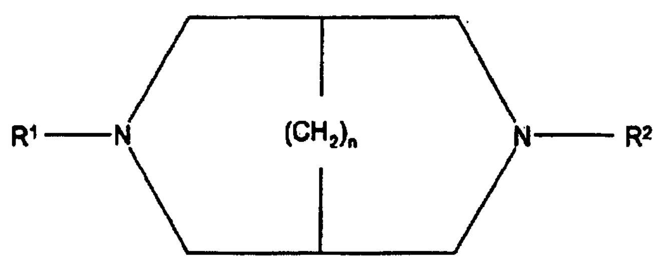

**(I)**

eines seiner Enantiomere oder eine Mischung von Enantiomeren oder ein pharmazeutisch annehmbares Salz davon, worin
n 1, 2 oder 3 bedeutet,
$R^1$ Wasserstoff oder Benzyl bedeutet, und
$R^2$ eine Pyrazinyl- oder Pyridazinylgruppe bedeutet, wobei diese heterocyclische Gruppe ein- oder mehrfach mit Substituenten substituiert sein kann, ausgewählt aus der Gruppe bestehend aus $C_{1-6}$-Alkyl, $C_{1-6}$-Alkoxy, Halogen, $CF_3$, Phenyl und Naphthyl.

**2.** Diazabicycloalkanderivat nach Anspruch 1, worin $R^2$ eine 2-Pyrazinylgruppe bedeutet, die optional in der 5- oder 6-Stellung mit einem Substituenten substituiert ist, ausgewählt aus der Gruppe bestehend aus $C_{1-6}$-Alkyl, Halogen, $CF_3$, Phenyl und Naphthyl.

**3.** Diazabicycloalkanderivat nach Anspruch 2, welches 3-Benzyl-7-(6-chlor-2-pyrazinyl)-3,7-diazabicyclo-[3.3.1]-nonan oder 3-*H*-7-(6-Chlor-2-pyrazinyl)-3,7-diazabicyclo-[3.3.1]-nonan,
eines seiner Enantiomere oder eine Mischung von Enantiomeren oder ein pharmazeutisch annehmbares Salz davon ist.

**4.** Diazabicycloalkanderivat nach Anspruch 1, worin $R^2$ eine 3-Pyridazinylgruppe bedeutet, die optional in der 5- oder 6-Stellung mit einem Substituenten substituiert ist, ausgewählt aus der Gruppe bestehend aus $C_{1-6}$-Alkyl, Halogen, $CF_3$, Phenyl und Naphthyl.

**5.** Diazabicycloalkanderivat nach Anspruch 4, welches
3-*H*-7-(6-Phenyl-3-pyridazinyl)-3,7-diazabicyclo-[3.3.1]-nonan,
3-Benzyl-7-(6-phenyl-3-pyridazinyl)-3,7-diazabicyclo-[3.3.1]-nonan,
3-Benzyl-7-(6-chlor-3-pyridazinyl)-3,7-diazabicyclo-[3.3.1]-nonan,
3-*H*-7-(6-Chlor-3-pyridazinyl)-3,7-diazabicyclo-[3.3.1]-nonan,
3-Benzyl-7-(6-chlor-3-pyddazinyl)-3,7-diazabicyclo-[3.3.2]-decan oder
3-Benzyl-7-(6-chlor-3-pyridazinyl)-3,7-diazabicyclo-[3.3.3]-undecan,
eines seiner Enantiomere oder eine Mischung von Enantiomeren oder ein pharmazeutisch annehmbares Salz davon ist.

**6.** Pharmazeutische Zusammensetzung, umfassend eine therapeutisch wirksame Menge des Diazabicycloalkanderivats nach einem der Ansprüche 1 bis 5 oder ein pharmazeutisch annehmbares Additionssalz davon, zusammen mit wenigstens einem pharmazeutisch annehmbaren Träger oder Verdünnungsmittel.

**7.** Verwendung des Diazabicycloalkanderivats gemäß einem der Ansprüche 1 bis 5 oder eines pharmazeutisch annehmbaren Salzes davon zur Herstellung einer pharmazeutischen Zusammensetzung zur Behandlung, Vorbeugung oder Erleichterung einer Krankheit oder einer Störung oder eines Zustandes eines Säugers, einschließlich des Menschen, wobei die Krankheit, die Störung oder der Zustand auf die Modulation von cholinergischen Rezeptoren und/oder Monoamin-Rezeptoren anspricht.

**8.** Verwendung gemäß Anspruch 7, worin die Krankheit, die Störung oder der Zustand sich auf das Zentralnervensystem bezieht.

**9.** Verwendung gemäß Anspruch 7, worin die Krankheit, die Störung oder der Zustand Angstzustände, Wahmehmungsstörungen, Lemschwäche, Gedächtnisschwäche und -störung, Alzheimersche Krankheit, Aufmerksamkeitsschwäche, Aufmerksamkeitsdefizit-Hyperaktivitätsstörung, Parkinsonsche Krankheit, Huntingtonsche Krankheit, amyotrophe Lateralsklerose, Gilles de la Tourette-Syndrom, Depression, Manie, manische Depression, Schizophrenie, obsessive kompulsive Störungen (OCD), Panikattacken, Essstörungen, wie Anorexia nervosa, Bulimie und Verstopfung, Narkolepsie, Schmerzempfindung, AIDS-Demenz, senile Demenz, periphere Neuropathie, Autismus, Dyslexie, tardive Dyskinesie, Hyperkinesie, Epilepsie, Bulimie, posttraumatisches Syndrom, Sozialphobie, Schlafstörungen, Pseudodemenz, Ganser-Syndrom, prämenstruelles Syndrom, Syndrom der späten Lutealphase, chronisches Ermüdungssyndrom, Mutismus, Trichotillomanie und Jetlag ist.

**10.** Verwendung gemäß Anspruch 7, worin die Krankheit, die Störung oder der Zustand mit Kontraktionen der glatten Muskeln, einschließlich konvulsive Störungen, Angina pectoris, vorzeitige Wehen, Krämpfe, Diarrhoe, Asthma, Epilepsie, tardive Dyskinesie, Hyperkinesie, vorzeitige Ejakulation und Erektionsschwierigkeiten, verbunden ist.

**11.** Verwendung gemäß Anspruch 7, worin die Krankheit, die Störung oder der Zustand mit dem endokrinen System, wie Thyreotoxikose, Pheochromozytom, Bluthochdruck und Arrhythmien, verbunden ist.

**12.** Verwendung gemäß Anspruch 7, worin die Krankheit, die Störung oder der Zustand eine neurodegenerative Störung ist, einschließlich vorübergehender Anoxie und induzierter Neurodegeneration.

**13.** Verwendung gemäß Anspruch 7, worin die Krankheit, die Störung oder der Zustand eine entzündliche Störung ist, einschließlich entzündliche Hautstörungen, wie Akne und Rosacea, Morbus Crohn, entzündliche Darmerkrankung, Colitis ulcerosa und Diarrhoe.

**14.** Verwendung gemäß Anspruch 7, worin die Krankheit, die Störung oder der Zustand sowohl schwacher, mäßiger oder selbst starker Schmerz von akutem, chronischem oder wiederkehrendem Charakter als auch durch Migräne,

postoperativem Schmerz oder Phantomgliedschmerz hervorgerufener Schmerz ist.

**15.** Verwendung gemäß Anspruch 7, worin die Krankheit, die Störung oder der Zustand mit Entzugssymptomen verbunden ist, hervorgerufen durch die Beendigung der Verwendung von süchtig machenden Substanzen, einschließlich Nikotin enthaltende Produkte, wie Tabak, Opiate, wie Heroin, Kokain und Morphin, Benzodiazepine und Benzodiazepin-ähnliche Wirkstoffe und Alkohol.

## Revendications

**1.** Dérivé diazabicycloalcane représenté par la formule I

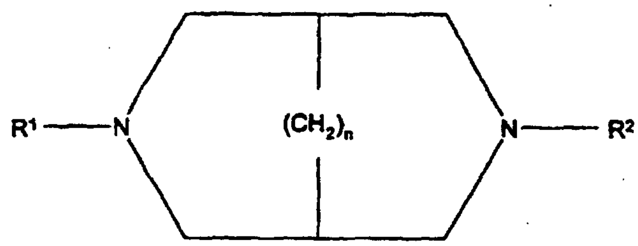

(I)

l'un quelconque de ses énantiomères ou un mélange quelconque de ses énantiomères, ou un de ses sels pharmaceutiquement acceptables,
dans laquelle,
n représente 1, 2 ou 3 ;
$R^1$ représente un hydrogène ou un groupe benzyle ; et
$R^2$ représente un groupe pyrazinyle ou pyridazinyle, lequel groupe hétérocyclique peut être substitué une ou plusieurs fois par des substituants choisis dans le groupe constitué par les groupes alkyle en $C_1$ à $C_6$, alcoxy en $C_1$ à $C_6$, halogène, $-CF_3$, phényle ou naphtyle.

**2.** Dérivé diazabicycloalcane selon la revendication 1, dans lequel $R^2$ représente un groupe 2-pyrazinyle qui est éventuellement substitué en position 5 ou 6 par un substituant choisi dans le groupe constitué par les groupes alkyle en $C_1$ à $C_6$, halogène, $-CF_3$, phényle ou naphtyle.

**3.** Dérivé diazabicycloalcane selon la revendication 2, qui est
le 3-benzyl-7-(6-chloro-2-pyrazinyl)-3,7-diazabicyclo-[3.3.1]-nonane ; ou
le 3-*H*-7-(6-chloro-2-pyrazinyl)-3,7-diazabicyclo-[3.3.1]-nonane ;
l'un quelconque de leurs énantiomères ou un mélange quelconque d'énantiomères, ou un de leurs sels pharmaceutiquement acceptables.

**4.** Dérivé diazabicycloalcane selon la revendication 1, dans lequel $R^2$ représente un groupe 3-pyrazinyle qui est éventuellement substitué en position 5 ou 6 par un substituant choisi dans le groupe constitué par les groupes alkyle en $C_1$ à $C_6$, halogène, $-CF_3$, phényle ou naphtyle.

**5.** Dérivé diazabicycloalcane selon la revendication 4, qui est
le 3-*H*-7-(6-phényl-3-pyridazinyl)-3,7-diazabicyclo-[3.3.1]-nonane ;
le 3-benzyl-7-(6-phényl-3-pyridazinyl)-3,7-diazabicyclo-[3.3.1]-nonane ;
le 3-benzyl-7-(6-chloro-3-pyridazinyl)-3,7-diazabicyclo-[3.3.1]-nonane ;
le 3-*H*-7-(6-chloro-3-pyridazinyl)-3,7-diazabicyclo-[3.3.1]-nonane ;
le 3-benzyl-7-(6-chloro-3-pyridazinyl)-3,7-diazabicyclo-[3.3.2]décane ; ou
le 3-benzyl-7-(6-chloro-3-pyridazinyl)-3,7-diazabicyclo-[3.3.3]undécane ;
l'un quelconque de ses énantiomères ou un mélange quelconque de ses énantiomères, ou un de ses sels pharmaceutiquement acceptables.

**6.** Composition pharmaceutique comprenant une quantité thérapeutiquement efficace d'un dérivé diazabicycloalcane selon l'une quelconque des revendications 1 à 5, ou d'un de ses sels d'addition pharmaceutiquement acceptables, conjointement avec au moins un véhicule ou un diluant pharmaceutiquement acceptable.

**7.** Utilisation du dérivé diazabicycloalcane selon l'une quelconque des revendications 1 à 5, ou d'un de ses sels d'addition pharmaceutiquement acceptables, pour fabriquer une composition pharmaceutique pour le traitement, la prévention ou le soulagement d'une maladie ou d'un trouble ou d'un état chez un mammifère, y compris un être humain, laquelle maladie, lequel trouble ou lequel état est sensible à la modulation des récepteurs cholinergiques et/ou monoamine.

**8.** Utilisation selon la revendication 7, dans laquelle la maladie, le trouble ou l'état concerne le système nerveux central.

**9.** Utilisation selon la revendication 7, dans laquelle la maladie, le trouble ou l'état est l'anxiété, les troubles cognitifs, les troubles de l'apprentissage, les troubles et le dysfonctionnement de la mémoire, la maladie d'Alzheimer, le déficit de l'attention, l'hyperactivité avec déficit de l'attention, la maladie de Parkinson, la maladie d'Huntington, la sclérose latérale amyotrophique, le syndrome de Gilles de la Tourette, la dépression, la manie, la psychose maniaco-dépressive, la schizophrénie, les troubles obsessionnels compulsifs (TOC), les troubles paniques, les troubles alimentaires tels que l'anorexie, la boulimie et l'obésité, la narcolepsie, la nociception, la démence du sida, la démence sénile, la neuropathie périphérique, l'autisme, la dyslexie, la dyskinésie tardive, l'hyperkinésie, l'épilepsie, la boulimie, le syndrome post-traumatique, la phobie sociale, les troubles du sommeil, la pseudo-démence, le syndrome de Ganser, le syndrome prémenstruel, le syndrome de phase lutéale tardive, le syndrome de fatigue chronique, le mutisme, la trichotillomanie et les troubles dus à un décalage horaire.

**10.** Utilisation selon la revendication 7, dans laquelle la maladie, le trouble ou l'état est lié aux contractions des muscles lisses, comprenant les troubles convulsifs, l'angine de poitrine, l'accouchement prématuré, les convulsions, la diarrhée, l'asthme, l'épilepsie, la dyskinésie tardive, l'hyperkinésie, l'éjaculation précoce et les troubles de l'érection.

**11.** Utilisation selon la revendication 7, dans laquelle la maladie, le trouble ou l'état concerne le système endocrinien comme la thyréotoxicose, le phéochromocytome, l'hypertension et l'arythmie.

**12.** Utilisation selon la revendication 7, dans laquelle la maladie, le trouble ou l'état est un trouble neuro-dégénératif, y compris l'anoxie transitoire ou la neuro-dégénérescence induite.

**13.** Utilisation selon la revendication 7, dans laquelle la maladie, le trouble ou l'état est un trouble inflammatoire, comprenant les affections cutanées inflammatoires telle que l'acné et l'acné rosacé, la maladie de Crohn, les maladies inflammatoires intestinales, la recto-colite hémorragique et la diarrhée.

**14.** Utilisation selon la revendication 7, dans laquelle la maladie, le trouble ou l'état est une douleur légère, modérée ou même intense de caractère aigu, chronique ou récurrent, ainsi qu'une douleur provoquée par une migraine, une douleur postopératoire et une douleur du membre fantôme.

**15.** Utilisation selon la revendication 7, dans laquelle la maladie, le trouble ou l'état est lié à des symptômes de sevrage provoqué par l'arrêt de l'utilisation de substances addictives, comprenant les produits contenant de la nicotine tels que le tabac, les opioïdes tels que l'héroïne, la cocaïne et la morphine, les benzodiazépines et les médicaments analogues aux benzodiazépines et l'alcool.